# EUROPÄISCHE PATENTANMELDUNG

(11) **EP 1 068 804 A1**
(43) Veröffentlichungstag der Anmeldung: **17.01.2001**
(21) Anmeldenummer: 00112564.0
(22) Anmeldetag: 14.06.2000
(51) Int. Cl.: A23K 1/16

(54) **Verfahren zur Herstellung und Verwendung von Lysinbase-Trockenpulvern**

(30) Priorität: 12.07.1999 DE 19932059
(71) Anmelder: BASF AKTIENGESELLSCHAFT, 67056 Ludwigshafen (DE)
(72) Erfinder: Choi, Jong Soo, Seoul Korea (KR); Kim, Tae Hui, Kunsan, Jeonbuk Korea (KR); Kim, Sung Hyeon, Kunsan, Jeonbuk Korea (KR); Du, Yun Su, Kunsan, Jeonbuk Korea (KR); Eidelsburger, Ulrich, Dr., 67149 Meckenheim (DE); Meyer, Joachim, Dr., 67133 Maxdorf (DE)

(57) **Zusammenfassung**

Die Erfindung betrifft ein Futtermittel oder Prämix enthaltend Lysinbase-Trockenpulver, wobei die Trockenmasse einen Anteil an freier Lysin-Base größer 70 Gew.-% enthält, sowie ein Verfahren zur Herstellung von Lysin-Base-Trockenpulver und dessen Verwendung.

## Beschreibung

Die Erfindung betrifft die Verwendung von Lysinbase-Trockenpulvern in Nahrungsmitteln, pharmazeutischen Zubereitungen und Tierfuttermitteln sowie ein Verfahren zu ihrer Herstellung.

L-Lysin findet als essentielle Aminosäure eine breite Anwendung als Zusatz in Nahrungsmitteln und Futtermitteln, beispielsweise zur Herstellung diätetischer Lebens- und Futtermittel. Auch in der Medizin als Bestandteil von Infusionslösungen werden Aminosäuren, wie Lysin breit angewendet. L-Lysin fördert beispielsweise das Knochenwachstum und regt die Zellteilung und Nucleosidsynthese an.

Insgesamt wird über 90 % des weltweit synthetisierten L-Lysins für die Herstellung von Futtermitteln, speziell für die Herstellung von Schweine- oder Geflügelfutter verwendet. Die Verwendung von L-Lysin zur Futterherstellung dient der bedarfsgerechten Versorgung der Tiere mit der in üblichen in der Tierernährung verwendeten Proteinquellen im Unterschuß vorhandenen essentiellen Aminosäure. Bei Verfütterung von Lysin wird die Limitierung aufgehoben und die zootechnischen Leistungen der Tiere können deutlich gesteigert werden.

Zur Supplementierung von Futtermitteln, z.B. mit L-Lysin wird bisher überwiegend das L-Lysin-Monohydrochlorid (L-Lysin-HCl) mit einem L-Lysingehalt von ca. 79 % eingesetzt. Die Verwendung von Lysin-Hydrochlorid hat zur Folge, daß die Tiere verstärkt Wasser aufnehmen (US 4,919,945). Dadurch kann es häufig zu dem unerwünschten "wet litter" und damit zu einer erhöhten Infektionsgefahr der Tiere kommen. Um zu einem weniger hygroskopischen Produkt zu gelangen, mußte in der Vergangenheit eine große Menge an Zuschlagsstoffen in das Konzentrat eingemischt werden. Dadurch wurde aber der in vielen Fällen ohnehin schon relativ geringe Gehalt der Aminosäure noch weiter vermindert.

Weiterhin haben basische Aminosäuren in Form ihrer Chloridsalze einen sehr bitteren Geschmack, der bei höheren Zulagen zum Futter zu einer geringeren Nährstoffaufnahme durch die Tiere führt.

Die Verwendung von L-Lysin-Hydrochlorid in der Medizin kann zu einer hyperchlorämischen metabolischen Acidose in Infusionslösungen führen. Die Verwendung derartiger Infusionslösungen bei Patienten, die an Niereninsuffizienz leiden, führt zu einer unerwünschten weiteren Belastung des schon gestörten Elektrolythaushaltes des Patienten durch die Chloridionen.

Aus dem Stand der Technik (EP-B 122 163) ist ein Verfahren bekannt, nach dem bei Einhaltung ganz spezieller Fermentationsbedingungen eine rohe Fermentationsbrühe erhalten werden kann, die sich zu einem festen und stabilen Produkt eintrocknen läßt. Dieses Produkt, das durch Entwässerung der gesamten Fermentationsbrühe erhalten werden kann, hat jedoch nur einen L-Lysin-Gehalt von 35 bis 48 Gewichtsprozent und liegt demnach deutlich niedriger als der des L-Lysin-Monohydrochlorids.

In EP-B 533 039 wird ein Tierfuttermittelsupplement mit hohem Gehalt einer "Aminosäure" beansprucht, das noch den überwiegenden Teil der Inhaltsstoffe der Fermentationsbrühe enthält. Mit dem offebarten Fermentationsverfahren (s. Ausführungsbeispiele) erhält man als Endprodukte nicht die freien Aminosäuren, sondern die Säuren in Form ihrer Salze, z.B. als Sulfate oder Carbonate, wie aus den Fermentationsbedingungen hervorgeht.

Die Fermentationsbrühe wird ohne eine weitere Reinigung sprühgetrocknet. Bei den in der Patentschrift angegebenen Gewichtsprozenten an Aminosäure, z.B. an L-Lysinbase, handelt es sich also nur um einen, ausgehend vom Lysinsalz, theoretisch berechneten Wert an freier Base.

Als weitere Produktform wird eine flüssige Lysinbase hergestellt, die neben 50 % Lysinbase Fermentationsnebenprodukte und hauptsächlich Wasser enthält. Neben hohen Transport-, Lager-, Verpackungs- und Handlingskosten aufgrund des hohen Gesamtvolumens, hat flüssige Lysinbase den Nachteil, daß es spezieller Applikationstechniken bedarf und auch hier bei der Herstellung die Wertproduktkonzentration gering ist.

Für viele Lysinformen im getrockneten Zustand wird eine produkteigene Klebrigkeit (Stickyness) beschrieben, zu deren Verminderung im Stand der Technik bestimmte Zusätze, beispielsweise CaSO₄, beschrieben sind (DE-A 2447274).

Aufgabe der vorliegenden Erfindung war es, ein L-Lysinbase-Trockenpulver mit einem hohen Gehalt an freiem L-Lysin zur Verfügung zu stellen, welches eine hohe nutritive Wirkung aufweist sowie ein Verfahren zu dessen Herstellung. Das feste Endprodukt sollte gute Lager- und Verarbeitungseigenschaften aufweisen. Des weiteren sollte das Produkt eine geringe Klebrigkeit aufweisen, so daß Zusätze zu deren Herabsetzung vermieden werden, dadurch die Wertproduktkonzentration unnötig herabgesetzt würde.

Es wurde nun gefunden, daß diese Aufgabe durch ein Futtermittel oder Prämix, enthaltend Lysin-Base-Trockenpulver, dadurch gekennzeichnet, daß die Trockenmasse einen Anteil an freier Lysin-Base größer als 70 Gew.-% enthält, gelöst werden kann.

Das erzeugte Produkt ist vergleichbar zu Lysinsalzen (z.B. Hydrochlorid) verwendbar und zeichnet sich durch excellente Fließeigenschaften sowie durch geringe Klebrigkeit aus. Es kann weiterhin dazu benutzt werden, um durch Auflösen in Wasser erneut eine konzentrierte Lysin-Base-Lösung zu erzeugen.

Üblicherweise enthält das Produkt einen Restwassergehalt von ca. 2 bis 3 Gew.-%.

Der Anteil an freier Lysin-Base in der Trockenmasse ist größer als 70 Gew.-%, bevorzugt größer als 90 Gew.-%.

Lysinquellen für die Herstellung von L-Lysinbase-Trockenpulvern sind beispielsweise Proteine aus denen über Hydrolyse mit Säuren L-Lysin gewonnen werden kann. Über die chemische Synthese beispielsweise aus D,L-α-Amino-ε-caprolactam sind D,L-Lysin und durch anschließende Racematspaltung auch L-Lysin und D-Lysin zugänglich.

Üblicherweise wird L-Lysin über ein fermentatives Verfahren mit Hilfe von Mikroorganismen im wesentlichen der Gattungen Corynebacterium, Brevibacterium, Arthorbacter, Microbacterium, Bacillus oder Nordica und einer anschließenden Aufreinigung bevorzugt über einen Ionentauscher gewonnen. Dabei kann die Fermentationsbrühe direkt oder nach Abtrennung der Biomasse auf den Ionentauscher gegeben werden. Mikrobiologische Verfahren zur Herstellung von L-Lysin werden beispielsweise in *Trends of Biotechnology* 1983, *1*, 70-74 beschrieben.

Das erfindungsgemäße Verfahren zeichnet sich dadurch aus, daß die Trockenmasse einen Anteil an freier Lysin-Base größer 70 Gew.-%, bevorzugt größer 90 Gew.-% enthält.

Die erfindungsgemäßen Lysin-Base-Trockenpulver wurden dann über übliche Aufkonzentrierungs- und/oder Trocknungsverfahren wie z.B. Sprühtrocknung, Fluidized Spray Drying, Walzentrocknung oder Gefriertrocknung in Pulverform überführt. Bevorzugt wird als Trocknungsverfahren die Sprühtrocknung (EP-A 0 457 075;
EP-A-0 497 177), dabei beträgt die Temperatur 80 bis 200°C, bevorzugt 110 bis 170°C.

Der Ausdruck Lysin-Base bezeichnet das Lysin, vorzugsweise L-Lysin in Form der freien Base (H₂N-(CH₂)₄-CHNH₂-CO₂H; pK-Wert ∼ 10,5).

Neben dem Anteil an freier Lysin-Base (70 bis 97 Gew.-%) können noch geringe Anteile anderer Aminosäuren oder Proteine aus dem Fermentationsmedium Bestandteil der Trockenmasse sein.

Die Lysin-Base-Trockenpulver werden nach der erfindungsgemäßen Verwendung vorteilhaft in Nahrungsmitteln, bevorzugt in der Tierernährung eingesetzt, besonders bevorzugt als Zusatz in Futter für Schweine, Ferkel und Geflügel, wie z.B. Legehennen, Broiler oder Puten. Dabei können sie allen üblichen Tierfuttern wie beispielsweise Mischfutter und/oder Einzelfutter wie Futtergetreide wie Weizen, Hafer, Roggen, Gerste sowie Hülsenfrüchte, Mais oder "Corn-cob-mix" zugesetzt werden. Das Lysin-Base Trockenpulver kann den Futtermitteln direkt zugesetzt werden oder in Form von sogenannten "Prämixen", in denen sie mit anderen Futterzusatzstoffen vor Anwendung abgemischt werden.

Als Hilfsstoffe können den Lysin-Base-Trockenpulvern alle in der Landwirtschaft üblichen Hilfsstoffe zugegeben werden, wie Aromastoffe, Farbstoffe, Appetitanreger, Antibiotika, Probiotika und/oder Enzyme. Die Hilfsstoffe werden vorteilhafterweise in einer Menge von 0,1 Gew.-% bis 50 Gew.-% bezogen auf das Gewicht der Lysin-Base-Trockenpulver zugegeben, bevorzugt in einer Menge von 0,1 bis 10 Gew.-%.

Je nach Zusammensetzung des Futtermittels sind unterschiedliche Aufwandmengen der Lysin-Base-Trockenpulver vorteilhaft. In der Regel ist ein Zusatz von 0,1 bis 10 kg pro Tonne Tierfutter ausreichend für eine Lysinsupplementierung. Bevorzugt werden Lysinbasen in Mengen von 0,5 bis 5 kg pro Tonne Tierfutter zugesetzt.

Die Lysin-Base-Trockenpulver können zur Herstellung der Futtermittel oder Futtermittelgemische in an sich bekannterweise verwendet werden. Der Zusatz zum Futtermittel kann direkt nach der Ernte oder nach Lagerung erfolgen. Die feste oder flüssige Lysin-base kann dem Futtermittel direkt oder in Form von Prämixen vorteilhafterweise während der Pelletierung oder Extrusion des Futters über beispielsweise eine Dosierungseinrichtung zugesetzt werden.

Auch in pharmazeutischen oder kosmetischen Zubereitungen können die Lysin-Base- Trockenpulver verwendet werden.

Eine weitere Verwendung des erfindungsgemäßen Lysin-Base-Trockenpulvers ist der Einsatz zur technischen Synthese von Polylysin.

Die nachfolgenden Beispiele sollen die Erfindung näher erläutern:

### Beispiel 1: Fermentative Herstellung von L-Lysin

Nach einer konventionellen Lysinfermentation (72 h) mit Corynebacterium glutamicum ATCC 21526, wie in US 3,708,395 beschrieben wurde die Fermentationsbrühe mit H₂SO₄ auf pH 2 angesäuert, über einen Ionentauscher mit NH₃ eluiert, unter Vakuum von NH₃ befreit und auf 68 % Trockenmasse aufkonzentriert. Dieses Konzentrat wurde zur Sprühtrocknung benutzt.

### Beispiel 2: Sprühtrocknung von L-Lysin

Das Konzentrat aus Beispiel 1 wurde einer Sprühtrocknung unterzogen, die unter folgenden Bedingungen durchgeführt wurde:

### Sprühtrockner mit Atomizer

- Eingangstemperatur 165°C
- Ausgangstemperatur 110°C
- Atomizer Geschwindigkeit 10 000 rpm (Sprühtrockner: Niro Production minor)

Das derart getrocknete Produkt enthielt 97 % Trockenmasse und einen Lysin-Base-Anteil von 91,8 %. Es war nicht klebrig und ließ sich gut verarbeiten.

### Beispiel 3: Fütterungsversuch mit Lysinbase zur Wirksamkeit von Lysinbase 90 % in der Ferkelaufzucht

Im vorliegenden Versuch mit Absetzferkeln sollte das Versuchsprodukt Lysinbase 90 % auf seine Lysinwirksamkeit im Vergleich zu Sewon L-Lysin HCl 99 % Feed (79 % L-Lysin) getestet werden. Bei dem Versuchsprodukt handelt es sich um sprühgetrocknete Lysinbase mit einem Wirkstoffgehalt von 90 % L-Lysin.

Insgesamt 45 Ferkel (DL x P) mit einem Alter von 28 Tagen und einer mittleren Lebendmasse von 7,2 kg wurden für den Fütterungsversuch von 49 Tagen verwendet. Die Tiere stammten aus 9 Würfen, wobei sie nach dem Prinzip der Blockbildung zufällig auf die 5 Behandlungen verteilt wurden. Folgende Versuchsvarianten wurden getestet:

| Gruppe | Lysinzulage % | Testsubstanz % |
|---|---|---|
| I | - | - |
| II | 0,12 | 0,152 % L-Lysin HCl |
| III | 0,24 | 0,304 % L-Lysin HCl |
| IV | 0,12 | 0,133 % Lysinbase 90 % |
| V | 0,24 | 0,266 % Lysinbase 90 % |

Gruppe I diente als Negativkontrolle und erhielt das Basisfutter ohne Lysinzulage. Bei Gruppe II und III wurde dem Basisfutter 0,12 bzw. 0,24 % Lysin in Form des Handelsproduktes Sewon L-Lysine HCl 99 % Feed (min. 79 % L-Lysin, Vertrieb: BASF Aktiengesellschaft Ludwigshafen) zugesetzt. In Gruppe IV und V wurden die entsprechenden Lysinäquivalente in Form der Testsubstanz Lysinbase 90 % supplementiert. Die Zusammensetzung des verwendeten lysinarmen Basisfutters ist Tabelle 1 zu entnehmen. Das Basisfutter enthielt 0,79 % Lysin und sollte ca. 60 % des Lysinbedarfs der Ferkel decken. Alle weiteren Aminosäuren waren bedarfsdeckend enthalten. Mit Ausnahme von Lysin entsprach das Futter den Bedarfsnormen für Ferkelaufzuchtfutter II nach Normtyp.

Die Tiere wurden in Einzelkäfigen in einem klimatisierten Versuchsstall gehalten. Die Raumtemperatur wurde im Verlauf des Versuches von 26°C kontinuierlich auf 22°C gesenkt bei einer relativen Luftfeuchtigkeit von ca. 55 %. Das pelletierte Futter wurde ad libitum vorgelegt. Nicht verzehrtes Futter wurde zweimal wöchentlich zurückgewogen. Die Lebendmasse der Tiere wurde wöchentlich festgestellt. Als Versuchsparameter wurden Lebendmassezuwachs, Futteraufnahme sowie Futterverwertung ermittelt.

Die Versuchsdaten wurden varianzanalytisch ausgewertet, mittels F-Test auf Signifikanz geprüft und die Mittelwerte der einzelnen Gruppen mit den Student-Newman-Keuls-Test miteinander verglichen. Signifikante Unterschiede zwischen den Gruppenmittelwerten sind durch unterschiedliche Hochbuchstaben gekennzeichnet.

In Tabelle 2 sind die Ergebnisse der Lebendmasseentwicklung, täglichen Zunahmen, Futteraufnahme und Futterverwertung zusammengestellt. Die Tiere der Gruppe I wiesen eine signifikant schlechtere Gewichtsentwicklung, Futteraufnahme und Futterverwertung auf als die Tiere der mit den Lysinprodukten supplementierten Gruppen II-V. Demnach lag eine deutliche Unterversorgung am Lysin im Basisfutter vor.

Die Zulage von 0,12 % bzw. 0,24 % Lysin in Form des Lysin-HCl steigerte den täglichen Zuwachs signifikant von 209 g/d auf 312 bzw. 349 g, entsprechend um 49 % bzw. 67 % gegenüber der Gruppe I. Der entsprechende Zusatz an Lysin in Form der Lysinbase verbesserte den täglichen Zuwachs auf 308 bzw. 355 g, d.h. um 47 % bzw. 70 % gegenüber der Negativkontrolle (Gruppe I). Die Wachstumserhöhung war somit in beiden Lysinzulagen bei beiden Lysinprodukten vergleichbar ausgeprägt.

Die Futteraufnahme wurde ebenfalls signifikant durch die steigende Lysinversorgung beeinflußt. Bei Zusatz von 0,12 % bzw. 0,24 % Lysin in Form von Lysin-HCl nahmen die Ferkel um 27 % bzw. 30 % nachweisbar mehr Futter auf als die Tiere der Kontrollgruppe. Die Tiere der Gruppen IV und V mit entsprechender Zulage an Lysin base wiesen eine um 25 % bzw. 33 % erhöhte Futteraufnahme im Vergleich zu der Gruppe I auf. Zwischen den beiden Lysinformen trat somit eine vergleichbare Erhöhung der Futteraufnahme auf, was auch auf eine gute Akzeptanz der Lysin base schließen läßt.

Die Futterverwertung war durch den Zusatz von 0,12 % bzw. 0,24 % Lysin in Form von Lysin-HCl und auch Lysinbase uni 15 % bzw. 22 % signifikant verbessert gegenüber der Negativkontrolle. Auch der Effekt der Zulagenhöhe konnte für beide Lysinformen abgesichert werden. Die beiden Lysinprodukte zeigen demnach in der Futterverwertung eine vergleichbare Wirksamkeit.

Der vorliegende Versuch ergab eine vergleichbare biologische Wirksamkeit für das Versuchsprodukt Lysin base 90 % im Vergleich zum Standardprodukt Lysin-HCl bei einem Vergleich auf Basis gleicher supplementierter Lysinmengen. Dies trifft für alle untersuchten zootechnischen Parameter tägliche Zunahmen, Futteraufnahme sowie die Futterverwertung zu. Aus den Ergebnissen läßt sich schlußfolgern, daß das neuentwickelte Versuchsprodukt Lysinbase 90 % mindestens ebenso wirksam ist wie das Standardprodukt Lysin-HCl.

**Tabelle 1**

| Zusammensetzung des verwendeten Basisfutters | |
|---|---|
| Komponenten | % |
| Weizen | 40,00 |
| Mais | 18,12 |
| Gerste | 6,00 |
| Sojaextraktionsschrot | 14,15 |
| Maiskleberfutter | 10,00 |
| Fleischknochenmehl | 3,00 |
| Weizenkleie | 2,10 |
| Luzernegrünmehl | 2,00 |
| Sojaöl | 2,00 |
| DL-Methionin | 0,05 |
| L-Threonin | 0,08 |
| Mineral-, Vitamin- und Spurenelementsupplement | 2,50 |
| Rohprotein | 17,70 |
| Umsetzbare Energie, MJ/kg | 13,10 |
| Calcium | 0,90 |
| Phosphor | 0,70 |

**Tabelle 2**

| Einfluß von L-Lysin HCl 99 % und Lysinbase 90 % auf Lebendmasse, tägliche Zunahmen, tägliche Futteraufnahme und Futterverwertung von Absetzferkeln | | | | | |
|---|---|---|---|---|---|
| Gruppe | I | II | III | IV | V |
| Lysinzulage, % | - | 0,12 | 0,24 | 0,12 | 0,24 |
| Lysinform | - | Lysin HCl | Lysin HCl | Lysin base | Lysin base |
| Anfangsmasse, kg | 7,19 ±0,92 | 7,21 ±0,93 | 7,20 ±0,72 | 7,19 ±0,72 | 7,20 ±0,99 |
| Endmasse, kg | 18,51b ±3,98 | 22,50a ±2,87 | 24,30a ±3,12 | 22,28a ±4,16 | 24,59a ±2,28 |
| Relativ, % | 100 | 122 | 131 | 120 | 133 |
| Zunahmen, g/d | 209b ±66 | 312a ±44 | 349a ±38 | 308a ±50 | 355a ±72 |
| Relativ, % | 100 | 149 | 167 | 147 | 170 |
| Futteraufnahme, g/d | 465b ±121 | 592a ±83 | 606a ±64 | 583a ±107 | 619a ±99 |
| Relativ, % | 100 | 127 | 130 | 125 | 133 |
| Futterverwertung | 2,23a ±0,15 | 1,90b ±0,09 | 1,74c ±0,11 | 1,89b ±0,08 | 1,75c ±0,12 |
| Relativ, % | 100 | 85 | 78 | 85 | 78 |

## Patentansprüche

1. Futtermittel oder Prämix enthaltend Lysin-Base-Trockenpulver, dadurch gekennzeichnet, daß die Trockenmasse einen Anteil an freier Lysin-Base größer 70 Gew.-% enthält.

2. Futtermittel oder Prämix gemäß Anspruch 1, dadurch gekennzeichnet, daß die Trockenmasse einen Anteil an freier Lysin-Base größer 90 Gew.-% enthält.

3. Verfahren zur Herstellung von Lysin-Base-Trockenpulver, dadurch gekennzeichnet, daß die Trocknung so durchgeführt wird, daß die Trockenmasse einen Anteil an freier Lysin-Base größer 70 Gew.-% enthält.

4. Verfahren gemäß Anspruch 4, dadurch gekennzeichnet, daß die Lysinfermentationslösung aufgereinigt wird und die Lysinfermentationslösung ohne Neutralisation oder Salzbildung anschließend als freie Base sprühgetrocknet wird.

5. Verfahren gemäß Anspruch 4, dadurch gekennzeichnet, daß die Lysinfermentationslösung über Ionenaustauscher aufgereinigt wird und die Lysinfermentationslösung ohne Neutralisation oder Salzbildung anschließend als freie Base sprühgetrocknet wird.

6. Verfahren gemäß einem der Ansprüche 4 oder 5, dadurch gekennzeichnet, daß die Trockenmasse einen Anteil an freier Lysin-Base größer 90 Gew.-% enthält.

7. Verwendung von sprühgetrocknetem Lysin-Base-Trockenpulver, hergestellt gemäß einem der Ansprüche 4 bis 6, zur Herstellung oder Supplementierung eines Tierfuttermittels oder Prämixes.

8. Verwendung von sprühgetrockneten Lysin-Base-Trockenpulvern, hergestellt gemäß einem der Ansprüche 4 bis 6, als Einsatzstoff zur technischen Synthese von Polylysin.
